# FASCICULE DE BREVET EUROPEEN

(11) **EP 0 575 256 B1**
(45) Date de publication et mention de la délivrance du brevet: **07.11.2001**
(21) Numéro de dépôt: 93401561.1
(22) Date de dépôt: 17.06.1993
(51) Int. Cl.: G05B 19/12, A61M 5/172

(54) **Procédé d'assistance médicale au moyen d'un appareil de mesure et/ou de thérapie et dispositif de mise en oeuvre du procédé**
Verfahren zur medizinischen Hilfe mittels eines Messgerätes und/oder Therapie und Vorrichtung zum Gebrauch des Verfahrens
Medical aid procedure using a measuring apparatus and/or a therapy and means of implementing the procedure

(30) Priorité: 19.06.1992 FR 9207484
(43) Date de publication de la demande: 22.12.1993
(73) Titulaire: GEMPLUS, 13881 Gémenos Cédex (FR)
(72) Inventeur: Lassus, Bruno, Cabinet Ballot-Schmit, F-75116 PAris (FR)

(56) Documents cités:
- EP-A- 0 346 685
- EP-A- 0 393 784
- DE-A- 4 104 814
- US-A- 4 676 776
- US-A- 4 942 534
- US-A- 5 104 374
- DATABASE WPIL Section Ch, Week 9001, Derwent Publications Ltd., London, GB; Class B04, AN 90-003062 & JP-A-1 284 755 (PATROMA RESARCH YG) 16 Novembre 1989

## Description

L'invention concerne un procédé d'assistance médicale au moyen d'un appareil de mesure et/ou de thérapie. Elle concerne également un dispositif de mise en oeuvre du procédé.

Les progrès générés par la miniaturisation des composants puis des appareils électroniques et électriques conduisent de plus en plus au développement d'appareils accessibles par des utilisateurs dans un périmètre lié à leur activité professionnelle, à leur moyen de transport voire à leur domicile, ou sur un lieu de villégiature.

Il est quasiment certain que dans les années à venir de nombreux et nouveaux appareils vont connaître la miniaturisation pour se rapprocher de l'utilisateur ou même être de taille suffisamment réduite pour être facilement transportables. Le secteur de la santé est un secteur extrêmement intéressé par le développement d'une miniaturisation des outils de traitement et de saisie concernant tous les appareillages de mesure et/ou de thérapie en vue de réaliser ce que l'on désigne couramment par "soins ambulatoires".

Cependant, de nombreux problèmes restent à régler. En effet, outre la miniaturisation des appareils restent à régler les problèmes de contrôle de l'utilisation de ces appareils par des personnes non-spécialistes puisqu'il s'agit des patients eux-mêmes et les problèmes liés aux conséquences d'une mauvaise utilisation ou d'une utilisation abusive de ces appareils par les patients.

Il reste également à règler les problèmes de perte du temps et de mauvaises manipulations que rencontre le personnel infirmier, problèmes qui sont dus à la multiplicité et à la spécificité des appareils.

Afin de mieux illustrer quelques problèmes rencontrés on peut citer les exemples suivants :

Lorsqu'un appareil de mesure du taux de glycémie est placé chez un patient le patient doit réaliser des mesures de son taux de glycémie de façon périodique sur une durée déterminée. Il n'est pas rare, dans ce cas, de trouver dans les enregistrements des valeurs parasites provenant de sujet de son entourage, qui, dans un intérêt de curiosité ont réalisé une mesure de leur taux de glycémie.

Pour citer un autre exemple dans le cas d'un outil de thérapie demandant un réglage de certaines valeurs de paramètres que doit réaliser le patient, il a été observé que certains patients ont tendance à augmenter un paramètre tel que la durée de la séance par exemple, l'intensité du courant ou le débit, soit de façon involontaire, soit, le plus souvent de façon volontaire dès qu'ils constatent une amélioration de leur santé. Ces modifications induisent des troubles pour l'appréciation des résultats par les médecins et peuvent également provoquer des lésions chez ces patients.

D'autre part, pour de nombreuses personnes, et principalement chez les personnes âgées, la mémorisation et la prise de conscience de l'importance du ou des moments de l'enregistrement de certains facteurs ou d'un traitement est.difficile.

Il est également très difficile voire impossible de pouvoir vérifier si une personne a bien respecté la thérapie qui lui a été prescrite, si elle a bien suivi le nombre de séances requises, sans en augmenter le nombre, par exemple.

On pourra se rapporter au document US 4,676,776 constitué par l'état de la technique le plus proche. Ce document décrit un appareil médical de délivrance d'un produit médical en fonction d'un profil de configuration enregistré dans une unité logique constituée par une mémoire électriquement programmable. La programmation de cette mémoire est faite par un ordinateur distinct de l'appareil médical et distant.

Il n'est pas prévu dans ce document de sécuriser l'utilisation de l'appareil médical de manière à empêcher une auto-médication ou une sur-médication ou l'utilisation par une personne étrangère de l'appareil.

Ces objectifs sont atteints par les parties caractérisantes des revendications indépendantes 1 et 5.

On pourra également se rapporter à l'état de la technique constitué par les documents JP 1284755, EP 346 685 et US 5,104,374

La présente invention permet de remédier à ces problèmes.

L'invention a pour objet un procédé d'assistance médicale au moyen d'un appareil de mesure et/ou de thérapie, principalement caractérisé en ce qu'il consiste à charger sur un support d'informations amovible dédié à un patient, un programme opérationnel adapté à ce patient, destiné à piloter le fonctionnement de l'appareil de façon automatique et à déclencher le fonctionnement de l'appareil par lecture de ce programme.

Selon une autre caractéristique du procédé, le programme opérationnel comprend une phase automatique d'initialisation du déroulement des opérations effectuées à partir de paramètres de réglage chargés au moment du chargement du programme.

La présente invention a également pour objet un dispositif d'assistance médicale comprenant un appareil de mesure et/ou de thérapie principalement caractérisé en ce qu'il comprend des moyens de réception d'un support d'informations amovible, des moyens de lecture et d'écriture d'informations sur ce support amovible, les informations comprenant un programme opérationnel de pilotage de l'appareil, des paramètres de réglage de l'appareil relatifs au patient, des paramètres de contrôle du déroulement des opérations, des moyens de comparaison et des moyens d'affichage.

Selon l'invention, un support d'information amovible sera délivré au patient et aura, au préalable, été programmé par un médecin ou toute autre personne habilitée.

Le support d'informations amovible est par exemple réalisé par une carte à puce. Ainsi, le patient devra introduire la carte à puce qui lui est remise, dans un lecteur-enregistreur de cartes à puce, placé dans l'appareil ou relié à celui-ci.

L'invention s'applique à tout appareil de mesure et/ou de thérapie. Elle s'applique par exemple à des appareils de mesure du taux de glycémie, de mesure de la pression artérielle ou de poids. Elle s'applique également à des appareils qui permettent d'appliquer un champ électrique à partir de deux électrodes que l'on plaque sur une partie du corps à traiter.

L'invention s'applique également à des appareils de dialyse.

Dans le cas où des réglages de certaines valeurs de paramètres sont nécessaires pour un appareil, il s'agit par exemple du réglage d'une fréquence ou de l'intensité ou d'une durée, ces paramètres seront alors chargés au moment du chargement du programme par le médecin. Le nombre de séances prescrites, leur durée, éventuellement l'heure à laquelle le patient devra s'y soumettre pourront également être enregistrés en tant que paramètres au moment de l'enregistrement du programme et ceci, toujours par le médecin traitant.

Il peut également être prévu que soient enregistrées, sur ce support amovible, l'identité du patient auquel le support est remis ainsi que celle du médecin qui a enregistré le programme opérationnel.

Il peut également être prévu de protéger l'ensemble des informations enregistrées sur ce support par un code d'accès secret connu du médecin.

D'autres particularités et avantages de l'invention apparaîtront clairement dans la description suivante présentée à titre d'exemples non limitatifs, en regard des figures annexées qui représentent :
- La figure 1, le schéma d'un appareil d'induction d'un champ électrique selon l'invention destiné à appliquer des courants sur une partie du corps d'un patient ;
- La figure 2 représente un schéma du dispositif conforme à l'invention sous forme de blocs fonctionnels.

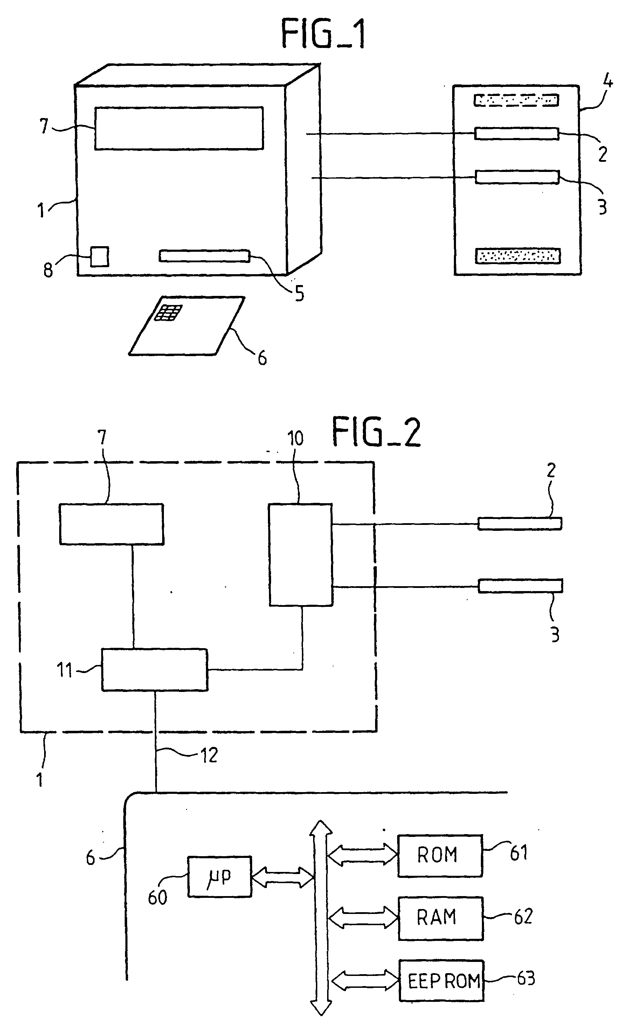

Le schéma de la figure 1 illustre, à titre d'exemple, un dispositif selon l'invention permettant à un patient d'appliquer des électrodes sur une partie de son corps afin de stimuler cette partie de corps selon la prescription médicale qui lui a été faite. Comme cela a été dit, l'invention s'applique, bien entendu, à d'autres types d'appareils. Elle peut s'appliquer notamment à des appareils de dialyse, elle peut également s'appliquer à des appareils de mesure, tels que des glucomètres ou des appareils de mesure de tension artérielle ou de poids.

Le dispositif comprend un appareil commercialisé sous la marque commerciale "Data-Vein" 1 auquel sont reliées deux électrodes 2-3 et des moyens d'attache 4 de ces électrodes de manière à ce que le patient puisse les placer au niveau de la partie du corps qu'il doit traiter. Selon l'exemple de réalisation qui va être décrit, l'appareil comprend des moyens de réception 5 d'un support d'informations amovible 6 de type carte à puce, par exemple, ainsi que des moyens de lecture et d'écriture d'informations sur le support qui seront décrits plus en détail en référence à la figure 2.

Bien entendu, selon une variante de réalisation, l'ensemble formé par les moyens de réception 5, et le lecteur-enregistreur pourrait être placé dans un boîtier en dehors de l'appareil et relié à ce dernier.

L'appareil 1 est également équipé d'un écran d'affichage 7 d'informations et d'une commande "marche", "arrêt" 8.

Il suffit que la patient introduise sa carte 6 dans l'appareil 1 pour que le programme qui a été, au préalable, chargé dans cette carte se déroule et permette de contrôler, par exemple, si l'heure de la séance est correcte, d'appliquer un courant d'intensité requise après la mise en place des électrodes, et cela pour une durée déterminée qui a été enregistrée en tant que paramètre lors de l'enregistrement du programme.

On se réfère maintenant au schéma de la figure 2. On retrouve sur cette figure les électrodes 2 et 3 qui sont reliées par des moyens classiques 10 de traitement et de transmission de signaux, ces moyens étant reliés à un circuit d'interface 11 lequel est relié, d'une part, à l'écran d'affichage 7 et d'autre part, au support d'informations amovible 6. Les moyens de liaison 12 sont par exemple réalisés par les contacts de la carte à puce dans le cas où ce support est réalisé par une carte à puce.

Comme cela a déjà été indiqué, le support d'informations amovible 6 peut être réalisé soit par une carte à puce, soit par un microcircuit électronique comprenant des mémoires et notamment, une mémoire de programme 61 dans laquelle a été enregistré le programme opérationnel permettant de piloter l'appareil. Ce support comporte également une mémoire de travail 62 du type "RAM". Le support peut comporter également une mémoire 63 de type programmable et effaçable électriquement (EEPROM) permettant, par exemple, d'enregistrer des paramètres pouvant être modifiés au cours du temps par le médecin qui a enregistré le programme opérationnel.

Le support amovible d'informations peut également comporter un microprocesseur 60 apte à gérer des opérations de lecture et d'écriture dans les différentes mémoires et à communiquer avec le circuit d'interface 11 de l'appareil. Il peut également être prévu d'avoir un microprocesseur apte à gérer des lectures et écritures des différentes mémoires de support amovible dans le circuit d'interface et non dans la carte.

L'interface 11 assure une adaptation entre les différentes commandes qu'elle reçoit et les signaux qu'elle doit transmettre aux éléments auxquels elle est reliée. Elle va donc permettre, par le biais des commandes du microprocesseur de transmettre les signaux de commande de l'écran d'affichage et de transmettre des signaux de commande au moyen de traitement et de transmission 10.

Le microprossesseur 60 assure l'exécution du programme opérationnel chargé dans la mémoire ROM 61 et d'une façon plus générale gère les lectures et écritures des mémoires, les signaux de commande d'affichage, les comparaisons entre mesures et paramètres enregistrés.

Lorsqu'un patient désire se soumettre à une séance thérapeutique, il introduit sa carte dans l'appareil et le programme opérationnel, chargé dans cette carte, commence à se dérouler. Ce programme va d'abord permettre de contrôler si le patient est encore autorisé à se soumettre à une séance, par comparaison du nombre de séances déjà enregistrées et du nombre autorisé (préalablement enregistré en tant que paramètre).

Lorsque la réponse est négative, le microprocesseur envoie un signal de commande d'arrêt de l'appareil.

Lorsque la réponse est positive, le programme continue à se dérouler et permet l'envoi d'un signal de commande par le microprocesseur, de manière à ce que le circuit de transmission délivre. des signaux d'amplitude requise pendant une durée déterminée. La fin de la séquence est enregistrée en mémoire sur la carte de manière à pouvoir décrémenter le nombre de séances encore admises.

L'ensemble des paramètres des réglages pour l'appareil sont, par exemple, constitués de l'intensité, de la fréquence et de la durée des signaux à appliquer. L'ensemble des paramètres de contrôle du déroulement des opérations sont constitués par le nombre de séances auxquelles devra se soumettre le patient, c'est-à-dire le nombre de mises en fonction de l'appareil autorisées, les instants autorisés d'utilisation et la durée d'utilisation.

Les mesures du nombre d'utilisations par le patient, des instants d'utilisation et des durées sont comparées aux paramètres préalablement enregistrés par le médecin et sont susceptibles d'être modifiés par ce dernier lors d'une consultation ultérieure, en fonction des résultats observés sur son patient.

Dans le cas d'un appareil de mesure, par exemple d'un glucomètre, toutes les mesures qui auront été réalisées à chaque séance prescrite, et contrôlées par le programme opérationnel seront enregistrées dans la mémoire de programme contenant des fichiers, à cet effet, de manière à pouvoir avoir un suivi médical simplement par lecture du résultat de ces mesures.

## Revendications

1. Procédé de fonctionnement d'un appareil médical (1) de mesure et/ou de thérapie consistant à :
- charger sur un support d'informations amovible dédié à un patient un programme opérationnel adapté à ce patient, destiné à piloter le fonctionnement de l'appareil de façon automatique ;
- charger des paramètres de réglage de l'appareil relatifs au patient, le programme opérationnel comprenant une phase automatique d'initialisation du déroulement des opérations effectuées à partir de ces paramètres de réglage ;
- charger des paramètres de contrôle du déroulement des opérations ;
- le déclenchement du fonctionnement de l'appareil se faisant par lecture de ce programme à l'insertion du support d' informations ;
**caractérisé en ce que** les paramètres de contrôle sont le nombre autorisé de mises en fonction de l'appareil, les instants autorisés d'utilisation, la durée autorisée d'utilisation,
et **en ce qu'**il consiste en outre à :
- effectuer des mesures des paramètres de contrôle ;
- comparer les mesures effectuées aux paramètres préalablement chargés ;
- verrouiller le démarrage ou arrêter l'appareil lorsqu'il y a discordance dans les comparaisons.

2. Procédé selon la revendication 1, **caractérisé en ce que** l'opération de chargement du programme opérationnel et des paramètres sur le support amovible est réalisée par un médecin ou toute personne habilitée disposant de moyens d'enregistrement du programme sur un tel support, le programme et les paramètres étant protégés par un code d'accès.

3. Procédé selon l'une quelconque des revendications précédentes, **caractérisé en ce qu'**il consiste à enregistrer également sur le support amovible l'identité du patient et celle du médecin ou de la personne habilitée qui charge le programme.

4. Procédé selon l'une quelconque des revendications précédentes, **caractérisé en ce qu'**il consiste à enregistrer les résultats de mesures en vue d'une exploitation.

5. Appareil médical de mesure et/ou de thérapie (1) comprenant :
- des moyens de réception (5) d'un support d'information amovible (6), ces informations comprenant un programme opérationnel de pilotage de l'appareil relatifs au patient, des paramètres de réglage de l'appareil relatifs au patient, des paramètres de contrôle du déroulement des opérations ;
- des moyens (60) de lecture et d'écriture d'informations sur ce support ;
- des moyens de déclenchement du fonctionnement de l'appareil à l'insertion du support par la lecture du programme opérationnel ;
- des moyens d'affichage des informations ;
**caractérisé en ce que** :
les paramètres de contrôle du déroulement des opérations sont le nombre autorisé de mises en fonction de l'appareil, les instants autorisés d'utilisation, la durée autorisée d'utilisation; et **en ce que** ledit appareil comporte des moyens pour effectuer des mesures du nombre des paramètres de contrôle ; des moyens de comparaison de ces mesures aux paramètres de contrôle pour commander le verrouillage du démarrage de l'appareil ou l'arrêt de l'appareil en cas de discordance dans les comparaisons.

6. Appareil selon la revendication 5, **caractérisé en ce qu'**il comprend au moins un microprocesseur (60) associé à des mémoires, implanté sur l'appareil ou sur le support amovible d'informations (6) ledit support étant du type carte à puce, pour réaliser les opérations précitées de lecture, d'écriture, de comparaison et d'affichage.

## Patentansprüche

1. Betriebsverfahren eines medizinischen Mess- und/oder Therapiegeräts (1), das darin besteht:
- auf einen herausnehmbaren, einen Patienten gewidmeten Informationsträger ein für diesen Patienten geeignetes operationelles Programm herunterzuladen, das den Betrieb des Geräts automatisch steuern soll;
- patientenbezogene Einstellparameter des Geräts herunterzuladen, wobei das operationelle Programm eine automatische Initialisierungsphase des Ablaufs der Operationen umfasst, die anhand dieser Einstellparameter durchgeführt werden;
- Kontrollparameter des Ablaufs der Operationen herunterzuladen;
- wobei der Betrieb des Geräts durch Lesen dieses Programms beim Einfügen des Informationsträgers ausgelöst wird;
**dadurch gekennzeichnet, dass** es sich bei den Kontrollparametern um die zulässige Anzahl der Einschaltungen des Geräts, die zulässigen Benutzungszeitpunkte, die zulässige Benutzungsdauer handelt, und dass es im übrigen darin besteht:
- Messungen der Kontrollparameter durchzuführen;
- die durchgeführten Messungen mit den zuvor heruntergeladenen Parametern zu vergleichen;
- das Einschalten zu verriegeln oder das Gerät abzuschalten, wenn die Vergleiche nicht übereinstimmen.

2. Verfahren nach Anspruch 1, **dadurch gekennzeichnet, dass** die Herunterlade-Operation des operationellen Programms und der Parameter auf den herausnehmbaren Träger von einem Arzt oder einer anderen befugten Person durchgeführt wird, die über Aufzeichnungsmittel des Programms auf einem derartigen Träger verfügen, wobei das Programm und die Parameter durch einen Zugriffscode geschützt werden.

3. Verfahren nach einem der vorangehenden Ansprüche, **dadurch gekennzeichnet, dass** es darin besteht, auf dem herausnehmbaren Träger ebenfalls die Identität des Patienten und diejenige des Arztes oder der befugten Person, die das Programm herunterlädt, aufzuzeichnen.

4. Verfahren nach einem der vorangehenden Ansprüche, **dadurch gekennzeichnet, dass** es darin besteht, die Messergebnisse für eine Nutzung aufzuzeichnen.

5. Medizinisches Mess- und/oder Therapiegerät (1) mit:
- Aufnahmemitteln (5) eines herausnehmbaren Informationsträgers (6), wobei diese Informationen ein operationelles, patientenbezogenes Steuerprogramm des Geräts, patientenbezogene Einstellparameter des Geräts, Kontrollparameter des Ablaufs der Operationen umfassen;
- Lese- und Schreibmitteln (60) von Informationen auf diesem Träger;
- Einschaltmitteln des Betriebs des Geräts beim Einfügen des Trägers durch das Lesen des operationellen Programms;
- Anzeigemitteln der Informationen;
**dadurch gekennzeichnet, dass** es sich bei den Kontrollparametern um die zulässige Anzahl der Einschaltungen des Geräts, die zulässigen Benutzungszeitpunkte, die zulässige Benutzungsdauer handelt; und dass das besagte Gerät Mittel umfasst, um Messungen der Anzahl der Kontrollparameter durchzuführen; Vergleichsmittel dieser Messungen mit den Kontrollparametern, um das Verriegeln der Einschaltung des Geräts oder das Ausschalten des Geräts zu steuern, wenn die Vergleiche nicht übereinstimmen.

6. Gerät nach Anspruch 5, **dadurch gekennzeichnet, dass** es mindestens einen, Speichern zugeordneten Mikroprozessor (60) umfasst, der auf dem Gerät oder auf dem herausnehmbaren Informationsträger (6) angeordnet ist, wobei der besagte Träger vom Typ einer Chipkarte ist, um die vorgenannten Lese-, Schreib-, Vergleichs- und Anzeigeoperationen durchzuführen.

## Claims

1. An operating method for a medical measuring and/or therapy apparatus (1) consisting in:
- loading, onto a removable information carrier dedicated to a patient, an operational program adapted to this patient, intended to control the functioning of the apparatus automatically;
- loading apparatus regulating parameters relating to the patient, the operational program comprising an automatic phase for initialising the running of the operations effected from these regulating parameters;
- loading parameters for controlling the running of the operation;
- the functioning of the apparatus being triggered by reading this program when the information carrier is inserted;
**characterised in that** the control parameters are the authorised number of start-ups of the apparatus, the times at which use is authorised and the authorised duration of use,
and **in that** it also consists in:
- making measurements of the control parameters;
- comparing the measurements made with the previously loaded parameters;
- locking the start-up or stopping the apparatus when there is disagreement in the comparisons.

2. A method according to Claim 1, **characterised in that** the operation of loading the operational program and the parameters onto the removable carrier is effected by a doctor or any authorised person having means of recording the program on such a carrier, the program and the parameters being protected by an access code.

3. A method according to any one of the preceding claims, **characterised in that** it also consists in recording on the removable carrier the identity of the patient and that of the doctor or authorised person loading the program.

4. A method according to any one of the preceding claims, **characterised in that** it consists in recording the results of measurements with a view to use.

5. Medical measurement and/or therapy apparatus (1) comprising:
- means (5) of receiving a removable information carrier (6), this information comprising an operational program for controlling the apparatus relating to the patient, apparatus regulating parameters relating to the patient, parameters for controlling the running of the operations;
- means (60) of reading and writing information on this carrier;
- means of triggering the functioning of the apparatus on insertion of the carrier by the reading of the operational program;
- information display means;
**characterised in that**:
the parameters for controlling the running of the operations are the authorised number of start-ups of the apparatus, the times at which use is authorised and the authorised duration of use; and **in that** the said apparatus has means for making measurements of the number of control parameters; means of comparing these measurements with the control parameters in order to control the locking of the start-up of the apparatus or the stopping of the apparatus in the event of disagreement in the comparisons.

6. The apparatus according to Claim 5, **characterised in that** it comprises at least one microprocessor (60) associated with memories, located on the apparatus or on the removable information carrier (6), the said carrier being of the smart card type, in order to perform the aforementioned operations of reading, writing, comparison and display.
